(19) Europäisches Patentamt / European Patent Office / Office européen des brevets

(11) **EP 4 219 003 A1**

(12) **EUROPEAN PATENT APPLICATION**
published in accordance with Art. 153(4) EPC

(43) Date of publication:
**02.08.2023 Bulletin 2023/31**

(21) Application number: **21872246.0**

(22) Date of filing: **13.09.2021**

(51) International Patent Classification (IPC):
**B01J 23/887** (2006.01)    **B01J 37/00** (2006.01)
**C07B 61/00** (2006.01)    **C07C 27/14** (2006.01)
**C07C 45/34** (2006.01)    **C07C 47/22** (2006.01)
**C07C 51/25** (2006.01)    **C07C 57/05** (2006.01)

(52) Cooperative Patent Classification (CPC):
**B01J 23/887; B01J 37/00; C07B 61/00; C07C 27/14; C07C 45/34; C07C 47/22; C07C 51/25; C07C 57/04**

(86) International application number:
**PCT/JP2021/033592**

(87) International publication number:
**WO 2022/065116 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.09.2020 JP 2020159209**

(71) Applicant: **Nippon Kayaku Kabushiki Kaisha Tokyo 100-0005 (JP)**

(72) Inventors:
• **OKUMURA Shigeki**
  **Sanyoonoda-shi, Yamaguchi 757-8686 (JP)**
• **YASUDA Shogo**
  **Sanyoonoda-shi, Yamaguchi 757-8686 (JP)**

(74) Representative: **Gille Hrabal Partnerschaftsgesellschaft mbB Patentanwälte Brucknerstraße 20 40593 Düsseldorf (DE)**

(54) **CATALYST PRECURSOR, CATALYST USING SAME, PRODUCTION METHOD FOR COMPOUND AND PRODUCTION METHOD FOR CATALYST**

(57) A catalyst precursor represented by the following formula (1) having an average particle diameter (D50), which is a particle diameter at which a cumulative volume fraction is 50%, of 10 $\mu$m or more and 40 $\mu$m or less,

$$Mo_{a1}Bi_{b1}Ni_{c1}Co_{d1}Fe_{e1}X_{f1}Y_{g1}Z_{h1}O_{i1} \qquad (1)$$

where, Mo, Bi, Ni, Co and Fe represent molybdenum, bismuth, nickel, cobalt, and iron, respectively; X is tungsten or the like; Y is potassium or the like; and Z belongs to the 1st to 16th groups in the periodic table and represents at least one element selected from elements other than the above Mo, Bi, Ni, Co, Fe, X, and Y

**FIG. 1**

EP 4 219 003 A1

**Description**

TECHNICAL FIELD

The present invention relates to a novel catalyst that is highly active and allows for providing a target product in a high yield, and particularly to a catalyst enabling stable production in a high yield even in a region having high catalytic activity for oxidatively producing an unsaturated aldehyde, an unsaturated carboxylic acid, or a conjugated diene.

BACKGROUND ART

A method for producing a corresponding unsaturated aldehyde or unsaturated carboxylic acid with propylene, isobutylene, t-butyl alcohol or the like as a raw material, or a catalytic gas phase oxidation method for producing 1,3-butadiene from butenes are widely carried out industrially. The use of a composite metal oxide catalyst containing bismuth and molybdenum as main components is well known to those skilled in the art.

In particular, regarding the method for producing a corresponding unsaturated aldehyde or unsaturated carboxylic acid with propylene, isobutylene, t-butyl alcohol or the like as a raw material, many reports have been made on means for improving the yield (for example, Patent Literatures 1, 2, etc.). Even when improvement is made by the means described above, further improvement in yield is required in the production of a corresponding unsaturated aldehyde and/or unsaturated carboxylic acid by partial oxidation reactions of propylene, isobutylene, t-butyl alcohol, or the like. For example, the yield of a target product influences an amount to be used of propylene, isobutylene, t-butyl alcohol, or the like required for the production and greatly influences the production cost.

In addition, for the purpose of improving the productivity of the catalyst and reducing the cost for the catalyst, it is well known for those skilled in the art to use a spray dryer when drying a mother liquid obtained by mixing various catalyst raw materials in a preparation step. In the case of obtaining the composite metal oxide catalyst, one of methods other than drying using a spray dryer is evaporating the mother liquid to dryness or gelling the mother liquid. The semi-finished product after this step is in a thickened or solidified state, and this makes it difficult to handle the product in a production method on an industrial scale and tends to increase the cost for the catalyst.

In the drying step using a spray dryer, the catalyst can be improved by optimizing process control factors. For example, Patent Literature 3 discloses that mechanical strength is improved when the hollow particle proportion of a granular composite metal oxide catalyst for use in a fluidized bed is 23% or less. Further, Patent Literature 4 discloses that a molded catalyst having high mechanical strength is obtained by specifying the viscosity of a prepared liquid and the bulk density of a calcined body. However, these methods include the addition of silica sol or the use of a homogenizer and the addition or the use may increase the viscosity of the mother liquid in the preparation step to make it difficult to feed the mother liquid to the spray dryer. This results in lower productivity. That is, it is not clear what kind of physical properties of the catalyst precursor obtained by stably drying the mother liquid using a spray dryer without increasing the viscosity of the mother liquid during the preparation step, are important for achieving a high yield of the final catalyst.

CITATION LIST

PATENT LITERATURE

> Patent Literature 1: WO 2016/136882
> Patent Literature 2: JP2017-024009A
> Patent Literature 3: JP6621569B
> Patent Literature 4: JP6204862B

SUMMARY OF INVENTION

TECHNICAL PROBLEM

The present invention proposes a catalyst precursor that allows for providing a catalyst with high selectivity and high yield of a target product, and a catalyst using the catalyst precursor.

SOLUTION TO PROBLEM

According to the research of the present inventors, it has been found that when the average particle diameter of a catalyst precursor obtained by drying a mixed solution or slurry of catalytically active component raw materials is within a specific range, the performance of a final catalyst, especially the catalytic activity, is remarkably improved. Thus, the present invention has been completed.

That is, the present invention relates to the following 1) to 10).

1) A catalyst precursor represented by the following formula (1) having an average particle diameter (D50), which is a particle diameter at which a cumulative volume fraction is 50%, of 10 $\mu$m or more and 40 $\mu$m or less:

$$Mo_{a1}Bi_{b1}Ni_{c1}Co_{d1}Fe_{e1}X_{f1}Y_{g1}Z_{h1}O_{i1} \qquad (1)$$

where Mo, Bi, Ni, Co and Fe represent molybdenum, bismuth, nickel, cobalt, and iron, respectively; X is at least one element selected from tungsten, antimony, tin, zinc, chromium, manganese, magnesium, calcium, silicon, aluminum, cerium, and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, h1, and i1 represent the number of atoms of molybdenum, bismuth, nickel, cobalt, iron, X, Y, Z, and oxygen, respectively; when a1 = 12, 0 < b1 $\leq$ 7, 0 $\leq$ c1 $\leq$ 10, 0 < d1 $\leq$ 10, 0 < c1+d1 $\leq$ 20, 0 $\leq$ e1 $\leq$ 5, 0 $\leq$ f1 $\leq$ 2, 0 $\leq$ g1 $\leq$ 3, 0 $\leq$ h1 $\leq$ 5, and i1 is a value determined by an oxidation state of each element.

2) The catalyst precursor according to the above 1), in which a hollow particle proportion is 0.0% or more and 4.3% or less.

3) The catalyst precursor according to the above 1) or 2), in which a specific surface area is 5.0 m$^2$/g or more and 10.4 m$^2$/g or less.

4) A catalyst obtained by molding the catalyst precursor according to any one of the above 1) to 3).

5) A catalyst in which the catalyst precursor according to any one of the above 1) to 3) is carried on an inert carrier.

6) The catalyst according to the above 5), in which the inert carrier is silica and/or alumina.

7) The catalyst precursor or the catalyst according to any one of the above 1) to 6), in which the catalyst precursor or the catalyst is used for producing an unsaturated aldehyde compound and/or an unsaturated carboxylic acid compound.

8) A method for producing an unsaturated aldehyde compound and/or an unsaturated carboxylic acid compound using the catalyst precursor or the catalyst according to any one of the above 1) to 7).

9) The method for producing an unsaturated aldehyde compound and/or an unsaturated carboxylic acid compound the above 8), in which the unsaturated aldehyde compound is acrolein and the unsaturated carboxylic acid compound is acrylic acid.

10) A method for producing a catalyst using the catalyst precursor according to any one of the above 1) to 3).

ADVANTAGEOUS EFFECTS OF INVENTION

The catalyst precursor according to the present invention is very effective in improving catalytic activity and yield in a catalytic gas phase oxidation reaction.

BRIEF DESCRIPTION OF DRAWINGS

FIG. 1 illustrates micrographs of a catalyst precursor (hollow particles).
FIG. 2 illustrates micrographs of a catalyst precursor (non-hollow particles).
FIG. 3 illustrates binarized images of the micrographs of FIG. 1.
FIG. 4 illustrates diagrams indicating the outer shape of the catalyst precursor (hollow particles) with broken lines and recess portions with dot regions in the binarized image of FIG. 3.

DESCRIPTION OF EMBODIMENTS

The present invention particularly relates to a catalyst with high catalytic activity and a method for producing the catalyst. The catalyst is produced using the catalyst precursor, or the catalyst precursor can be used as an oxidation catalyst as it is, and thus, the catalyst precursor is also described as the present invention.

Average particle diameter (D50) is 10 $\mu$m or more and 40 $\mu$m or less

The catalyst precursor according to the present invention has an average particle diameter of 10 $\mu$m or more and 40 $\mu$m or less.

The average particle diameter can be measured by a known method, for example using the following apparatus. For example, the average particle diameter is determined by measuring a particle diameter distribution using a laser diffraction scattering particle size distribution measuring device (trade name "LMS-2000e", manufactured by Seishin Enterprise Co., Ltd.) and calculating a volume average (median diameter) thereof. That is, the median value in volume fraction. The measurement conditions are not limited as long as it is a general condition known to those skilled in the art. For example, a scattering range is 10 to 20%, a measurement time is 3 seconds, the snap number is 3,000, a sample material is Fraunhofer, a dispersion medium is water, a stirrer pump is set to 2,500 rpm, and ultrasonic waves are not applied. That is, the average particle diameter means the particle diameter at which the cumulative volume fraction is 50%, and may be expressed as D50 in this description. Similarly, the particle diameter at which the cumulative volume fraction is 10% is expressed as D10, and the particle diameter at which the cumulative volume fraction is 90% is expressed as D90. The measurement method may be either a wet method or a dry method, and the wet method is used in the present embodiment.

When the average particle diameter (D50) is outside the range of 10 $\mu$m or more and 40 $\mu$m or less, the performance of the catalyst may not be stable, and this may result in a decrease in catalytic activity.

The lower limit of the preferred range of the average particle diameter (D50) is 15 $\mu$m, more preferably 20 $\mu$m, and particularly preferably 23 $\mu$m. In addition, the upper limit is preferably 38 $\mu$m, more preferably 35 $\mu$m, and particularly preferably 32 $\mu$m. That is, the most preferred range of the average particle diameter is 23 $\mu$m or more and 32 $\mu$m or less.

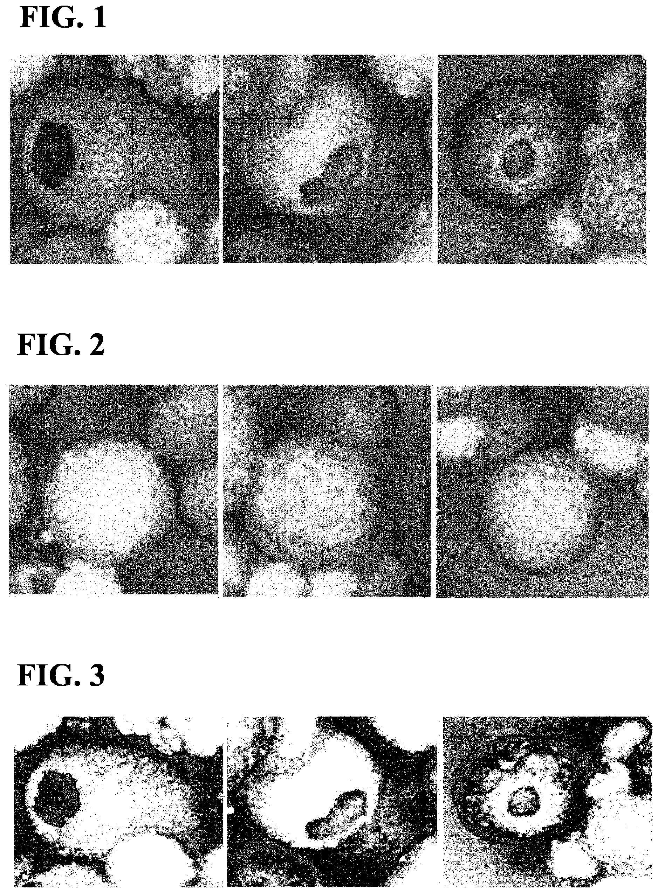

Hollow particle proportion of catalyst precursor is 0.0% or more and 4.3% or less The catalyst precursor according to the present invention preferably has a hollow particle proportion of 0.0% or more and 4.3% or less as defined below. That is, 1 g of a catalyst precursor sample is photographed with a microscope at a magnification of 20 times while carefully dispersing particles of the catalyst precursor sample so as not to overlap each other, and each particle of the obtained catalyst precursor sample is determined to be hollow or non-hollow. 200 or more particles of the catalyst precursor sample are measured, and the number of hollow particles in the catalyst precursor is divided by the number of the measured particles in the catalyst precursor and multiplied by 100 to obtain the hollow particle proportion of the catalyst precursor. In determination on whether a particle is hollow or non-hollow, a particle that is shaded by recesses or unevenness and in which the total area of the particle (total area in the portion appearing in the micrograph) exceeds 5% is regarded as hollow. Examples of the determination are shown in FIG. 1 and FIG. 2. The area proportions of the recesses and/or uneven portions to the catalyst particles in the catalyst precursor shown in order from the left in FIG. 1 are 11%, 12% and 6%, respectively. All of the catalyst precursors shown in FIG. 2 are determined to have no recesses. To determine hollow particles, increasing the contrast of a microscope image or binarizing the microscope image, or interpolating a portion where the catalyst particles to be measured overlap other catalyst particles as shown in the left in FIG. 1, or quantitatively calculating the area of the recesses or uneven portions can be performed using known image processing software, and the details thereof are not limited.

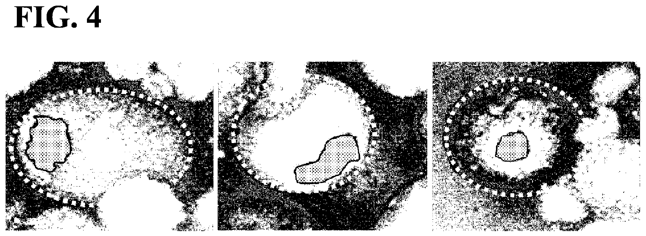

FIG. 3 illustrates binarized images of the micrographs of FIG. 1. FIG. 4 illustrates diagrams showing the outer shape of the catalyst precursor (hollow particles) with broken lines and recess portions with dot regions in the binarized image of FIG. 3. An example of a procedure for determining hollow particles will be described below with reference to FIG. 4. As shown in FIG. 4, the outer shape of the particle in the micrograph is assumed to be an ellipse (the ellipse indicated by the dashed line in FIG. 4), and the area of the ellipse drawn from five points on the outer periphery is taken as the total area of the particle. As for the recess portion (the portion indicated by the dot region in FIG. 4), the area of the recess portion is calculated by using commercially available image processing software after the outer edge of the recess portion is enclosed. In this case, the area of the recess portion (dot portion) is determined using Photoshop (registered trademark) manufactured by Adobe Inc.

The lower limit of the hollow particle proportion of the catalyst precursor is 0.1%, 0.3%, 0.5%, 1.0%, and 1.5% in a preferred order, and the upper limit of the hollow particle proportion of the catalyst precursor is 4.0%, 3.8%, 3.5%, 3.0%, 2.8%, 2.5% and 2.2% in a preferred order. That is, the most preferred range of the hollow particle proportion of the catalyst precursor is 1.5% or more and 2.2% or less.

Water content in catalyst precursor is 0.0 mass% or more and 15.0 mass% or less

The catalyst precursor according to the present invention preferably has a water content defined below of 0.0 mass% or more and 15.0 mass% or less. That is, the water content in the catalyst precursor is defined as a mass reduction rate (= 100 - (sample weight at 120°C) ÷ (charged sample weight) $\times$ 100 [mass%]) at 120°C when about 10 mg of the catalyst precursor is subjected to TG analysis and heated at a temperature rising rate of 1.5°C/min in an air atmosphere. The lower limit of the water content in the catalyst precursor is 0.1 mass%, 0.3 mass%, 0.5 mass%, 1.0 mass%, 1.5 mass%, 2.0 mass%, 2.5 mass%, 3.0 mass%, 4.0 mass%, 5.0 mass%, 6.0 mass%, 7.0 mass%, 8.0 mass%, 9.0 mass%, and 10.0 mass% in a preferred order, and the upper limit of the water content in the granules is 14.5 mass%, 14.0 mass%, 13.0 mass%, 12.0 mass%, and 11.0 mass% in a preferred order. That is, the most preferred range of the water content in the catalyst precursor is 10.0 mass% or more and 11.0 mass% or less.

The catalyst precursor according to the present invention is in a granule form before a step of molding the catalyst, is also described as preliminarily calcined powder in the present invention, and is obtained by granulating a slurry, which is obtained by mixing raw materials containing each element constituting the catalyst, using, for example, drum drying, spray drying, or evaporation to dryness, and then performing preliminary calcination. The drying method is particularly preferably spray drying in which the slurry can be dried into a granule within a short period of time.

The catalyst precursor according to the present invention preferably has a D10 of 10 μm or more and 25 μm or less. The lower limit of the D10 is 12 μm, 13 μm, 14 μm, and 15 μm in a preferred order. In addition, the upper limit thereof is 24 μm, 23 μm, 22 μm, 21 μm, 20 μm, 19 μm, and 18 μm in a preferred order. That is, the D10 is most preferably 15 μm or more and 18 μm or less.

In addition, the catalyst precursor according to the present invention preferably has a D90 of 40 μm or more and 65 μm or less. The lower limit of the D90 is 42 μm, 45 μm, 48 μm, and 50 μm in a preferred order. In addition, the upper limit thereof is 64 μm, 63 μm, 62 μm, 61 μm, and 60 μm in a preferred order. That is, the D90 is most preferably 50 μm or more and 60 μm or less.

In general, in order to change the parameters related to the catalyst precursor (the average particle diameter of the catalyst precursor, the hollow particle proportion of the catalyst precursor, and the water content in the catalyst precursor), the pH can be adjusted in the preparation step and the temperature or the like can be set in the drying step, and a particularly effective method is optimization of the rotation speed of a sprayer (atomizer) in the case of spray drying. The optimal rotation speed of the atomizer is influenced by the structure of the atomizer or the spray dryer, the temperature, pH and viscosity of the liquid to be dried, and the blending ratio of the catalyst constituents, and thus cannot be basically generalized. However, it is preferably 9,000 rpm or more and 22,000 rpm or less. The upper limit of the rotation speed of the atomizer is more preferably 20,000 rpm, particularly preferably 19,000 rpm, and most preferably 18,500 rpm. In addition, the lower limit of the rotation speed of the atomizer is more preferably 10,000 rpm, 12,000 rpm, and 14,000 rpm in order, particularly preferably 15,000 rpm, and most preferably 16,000 rpm. That is, the most preferred range of the rotation speed of the atomizer is 16,000 rpm or more and 18,500 rpm or less.

In addition, the rotation speed of the atomizer is also expressed by a relative centrifugal acceleration, and is preferably 12,500 G or more and 50,000 G or less. The lower limit of the elative centrifugal acceleration is more preferably 15,000 G, 17,500 G, 18,500 G, and 19,000 G in order, and the upper limit of the elative centrifugal acceleration is more preferably 45,000 G, 40,000 G, 37,500 G, 35,000 G and 32,500 G in order. That is, the most preferred range of the rotation speed of the atomizer is 19,000 G or more and 32,500 G or less.

Further, the inlet temperature and the outlet temperature of the sprayer during spray drying also influence the above parameters (the average particle diameter of the catalyst precursor, the hollow particle proportion of the catalyst precursor, the water content in the catalyst precursor). The inlet temperature is preferably 200°C or higher and 350°C or lower. The lower limit of the inlet temperature is more preferably 210°C, 220°C, 230°C, 250°C, 270°C, and 280°C in order, and the upper limit of the inlet temperature is more preferably 340°C, 330°C, 320°C, and 310°C in order. That is, the inlet temperature is most preferably 280°C or higher and 310°C or lower.

In addition, the outlet temperature is preferably 100°C or higher and 150°C or lower. The lower limit of the outlet temperature is more preferably 101°C, 102°C, 103°C, 104°C, and 105°C in order, and the upper limit of the outlet temperature is more preferably 140°C, 130°C, and 120°C in order. That is, the outlet temperature is most preferably 105°C or higher and 120°C or lower.

Further, the difference between the inlet temperature and the outlet temperature is preferably 50°C or higher and 140°C or lower. The lower limit of the difference is more preferably 70°C, 80°C, 90°C, 100°C, and 105°C in order, and the upper limit of the difference is more preferably 135°C, 130°C, 125°C, and 120°C in order. That is, the difference between the inlet temperature and the outlet temperature is most preferably 105°C or higher and 120°C or lower.

Further, the range of a parameter SD given by the following equation in a method for producing the catalyst precursor according to the present invention is preferably 5 or more and 41 or less. The lower limit of the range is more preferably 10, 15, 18, 20, and 21 in order, and the upper limit of the range is 40, 38, 36, and 35 in order. That is, the SD is most preferably 21 or more and 35 or less.

$$SD = 51.3 + 0.0766 \times \{(\text{inlet temperature of spray dryer, unit: °C}) - (\text{outlet temperature of spray dryer, unit: °C})\} - 0.00173 \times (\text{rotation speed of atomizer, unit: rpm})$$

Specific surface area

The catalyst precursor according to the present invention preferably has a specific surface area of 5.0 $m^2/g$ or more and 10.4 $m^2/g$ or less.

Here, the specific surface area of the catalyst precursor means the surface area per gram of the precursor, and can be measured by a method known to those skilled in the art. The details are not limited, and the specific surface area of the catalyst precursor can be measured by, for example, the following method. That is, a sample having a volume of 0.05 mL to 3.0 mL is charged into a sample tube having an inner diameter of 7 mm and subjected to a pretreatment at 300°C for 2 hours or longer. Then, a gas adsorption measuring device (Belsorp-mini (manufactured by MicrotracBEL Corp.)) is used to perform measurement after the nitrogen molecule diameter is set to 0.364 nm, the relative pressure ratio is set to 0.4, the adsorption temperature is set to -196°C, the measurement pore diameter range is set to 0.7 nm to 400 nm, and the adsorption gas is set to nitrogen. The measurement result is analyzed by a BET method to obtain the specific surface area value.

The lower limit of the preferred range of the specific surface area is 7.5, 8.0, 8.5, and 9.0 in a preferred order, and the upper limit thereof is 10.3, 10.2, 10.1, 9.8, and 9.5 in a preferred order. That is, the specific surface area is most preferably 9.0 $m^2/g$ or more and 9.5 $m^2/g$ or less.

In addition, the specific surface area of the catalyst, which is the final product obtained by the present application, is preferably 1.00 $m^2/g$ or more and 2.80 $m^2/g$ or less. The lower limit of the specific surface area of the catalyst is more preferably 1.20 $m^2/g$, 1.40 $m^2/g$, 1.60 $m^2/g$, 1.80 $m^2/g$, 2.00 $m^2/g$, and 2.20 $m^2/g$ in order, and the upper limit of the specific surface area of the catalyst is more preferably 2.70 $m^2/g$, 2.60 $m^2/g$, 2.50 $m^2/g$, and 2.40 $m^2/g$ in order. That is, the specific surface area of the catalyst is most preferably 2.20 $m^2/g$ or more and 2.40 $m^2/g$ or less.

Bulk density

The catalyst precursor according to the present invention preferably has a bulk density of 0.8 g/mL or more and 1.3 g/mL or less. The lower limit of the bulk density is more preferably 0.9 g/mL and 1.0 g/mL, and the upper limit of the bulk density is more preferably 1.2 g/mL and 1.1 g/mL. Therefore, the most preferred range is 1.0 g/mL or more and 1.1 g/mL or less.

Composition

The catalyst precursor according to the present invention preferably has a composition represented by the following formula (1).

$$Mo_{a1}Bi_{b1}Ni_{c1}Co_{d1}Fe_{e1}X_{f1}Y_{g1}Z_{h1}O_{i1} \qquad (1)$$

(In the formula, Mo, Bi, Ni, Co and Fe represent molybdenum, bismuth, nickel, cobalt, and iron, respectively; X is at least one element selected from tungsten, antimony, tin, zinc, chromium, manganese, magnesium, calcium, silicon, aluminum, cerium, and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1 g1, h1, and i1 represent the number of atoms of molybdenum, bismuth, nickel, cobalt, iron, X, Y, Z, and oxygen, respectively; when a1 = 12, $0 < b1 \leq 7$, $0 \leq c1 \leq 10$, $0 < d1 \leq 10$, $0 < c1+d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element)

In the above formula (1), when a1 = 12, preferred ranges of b1 to i1 are as follows.

The lower limit of b1 is 0.1, 0.2, 0.3, 0.4, and 0.5 in a preferred order, and the upper limit thereof is 6, 5, 4, 3, 2, and 1 in a preferred order. That is, the most preferred range of b1 is 0.5 or more and 1 or less.

The lower limit of c1 is 0.2, 0.5, 0.8, 1.0, 1.5, 1.8, 2.0, and 2.5 in a preferred order, and the upper limit thereof is 8.0, 7.0, 6.0, 5.0, 4.0, and 3.5 in a preferred order. That is, the most preferred range of c1 is 2.5 or more and 3.5 or less.

The lower limit of d1 is 1.0, 2.0, 3.0, 4.0, and 5.0 in a preferred order, and the upper limit thereof is 9.5, 9.0, 8.5, 8.0, 7.5, 7.0, 6.5, and 6.0 in a preferred order. That is, the most preferred range of d1 is 5.0 or more and 6.0 or less.

The lower limit of c1+d1 is 0.0, 2.0, 4.0, 6.0, 8.0, and 8.3 in a preferred order, and the upper limit thereof is 20.0, 15.0, 12.5, 11.0, 10.0, and 9.0 in a preferred order. That is, the most preferred range of c1+d1 is 8.3 or more and 9.0 or less.

The lower limit of e1 is 0.1, 0.2, 0.5, 0.8, 1.0, and 1.5 in a preferred order, and the upper limit thereof is 4.5, 4.0, 3.5, 3.0, 2.5, 2.0, 1.9, and 1.8 in a preferred order. That is, the most preferred range of e1 is 1.5 or more and 1.8 or less.

The upper limit of f1 is 1.8, 1.5, 1.0, 0.8, and 0.5 in a preferred order, and the lower limit thereof is preferably 0. That is, a more preferred range of f1 is 0 or more and 0.5 or less, and 0 is most preferred.

The lower limit of g1 is 0.01, 0.02, 0.03, 0.04, 0.05, 0.06, and 0.07 in a preferred order, and the upper limit thereof is 2, 1, 0.5, 0.4, 0.3, 0.2, and 0.1 in a preferred order. That is, the most preferred range of g1 is 0.07 or more and 0.1 or less.

The upper limit of h1 is 4.0, 3.0, 2.0, 1.8, 1.5, 1.0, 0.8, and 0.5 in a preferred order, and the lower limit thereof is preferably 0. That is, a more preferred range of h1 is 0 or more and 0.5 or less, and 0 is most preferred.

X in the formula (1) is preferably tungsten, antimony, zinc, magnesium, calcium, or cerium, and particularly preferably antimony or zinc.

Y in the formula (1) is preferably sodium, potassium, or cesium, more preferably potassium or cesium, and particularly preferably potassium.

Z in the formula (1) is preferably phosphorus.

The drying temperature is not limited as long as water can be removed, and may be normal temperature (25°C) when adjusting the pressure and time. However, in order to remove water more reliably and in a short period of time, 80°C or higher is preferred, and 90°C or higher is more preferred. When the pressure is not adjusted, the temperature is preferably 100°C or higher, and more preferably 150°C or higher.

For the preliminary calcination, calcination is performed at a temperature of about 200°C to 600°C for about 1 to 12 hours. The atmosphere during the calcination and the temperature rising rate are not limited as long as they are within the range known by those skilled in the art. For example, the atmosphere is an air atmosphere, an inert atmosphere such as nitrogen, or an organic compound-containing atmosphere containing more than 0% of organic compounds such as methanol and ethanol, and the temperature rising rate is 0.01°C/min or more and 10°C/min or less.

Carrying

The catalyst in which a preliminary calcined powder subjected to preliminary calcination after the preparation of the catalyst is carried on the inert carrier is particularly excellent as the catalyst of the present invention.

As the material of the inert carrier, known materials such as alumina, silica, titania, zirconia, niobia, silica alumina, silicon carbide, carbides, and mixtures thereof can be used. Further, the particle size, water absorption rate, mechanical strength, crystallinity of each crystal phase, mixing ratio, etc. are not limited, and an appropriate range of these should be selected in consideration of the final catalyst performance, molding properties, production efficiency, etc. The mixing ratio of the carrier and the preliminarily calcined powder is calculated as the active mass ratio according to the following equation based on the charged mass of each raw material.

$$\text{Active mass ratio (mass\%)} = (\text{mass of preliminary calcined powder used for molding})/\{(\text{mass of preliminary calcined powder used for molding}) + (\text{mass of carrier used for molding})\} \times 100$$

The upper limit of the active mass ratio is preferably 80 mass%, and more preferably 60 mass%.

The lower limit is preferably 20 mass%, and more preferably 30 mass%. That is, the most preferred range of the active mass ratio is 30 mass% or more and 60 mass% or less.

As the inert carrier, silica and/or alumina is preferable, and a mixture of silica and alumina is particularly preferable.

For the carrying, it is preferred to use a binder. Specific examples of the binder which can be used include water, ethanol, methanol, propanol, a polyhydric alcohol, polyvinyl alcohol as a polymer-based binder, and a silica sol aqueous solution as an inorganic binder; ethanol, methanol, propanol, and a polyhydric alcohol are preferred; a diol such as ethylene glycol and a triol such as glycerin is preferred; and an aqueous solution having a concentration of glycerin of 5 mass% or more is preferred. Using an appropriate amount of a glycerin aqueous solution allows for obtaining a high-performance catalyst having good molding properties and high mechanical strength. The amount of these binders used is usually 2 to 60 parts by mass with respect to 100 parts by mass of the preliminarily calcined powder. The amount of glycerin aqueous solution is preferably 10 to 30 parts by mass. The binder and the preliminarily calcined powder may be alternately or simultaneously supplied to a molding machine on the occasion of carrying.

Method for producing catalyst

A starting raw material for each element constituting the catalyst precursor or the catalyst according to the present invention is not limited. For example, as the molybdenum component raw material, molybdenum oxides such as molybdenum trioxide, molybdic acids or salts thereof such as molybdic acid, ammonium paramolybdate, and ammonium metamolybdate, and heteropolyacids containing molybdenum or salts thereof such as phosphomolybdic acid and silicomolybdic acid can be used.

As a raw material of a bismuth component, bismuth salts such as bismuth nitrate, bismuth carbonate, bismuth sulfate, and bismuth acetate, bismuth trioxide, metal bismuth and the like can be used. These raw materials can be used as solids or as an aqueous solution, a nitric acid solution, or a slurry of bismuth compounds generated from those aqueous solutions, and the nitrate, a solution thereof, or a slurry obtained from the solution is preferably used.

As a starting raw material for other constituent elements, ammonium salt, nitrate, nitrite, carbonate, subcarbonate, acetate, chloride, inorganic acid, inorganic acid salt, heteropolyacid, heteropolyacid salt, sulfate, hydroxide, organic acid salt, and oxide of metallic elements commonly used in this type of catalyst may be used, or a mixture thereof may be used in combination. Ammonium salts and nitrates are preferably used.

A compound containing these active components may be used alone or in combination of two or more. A slurry liquid can be obtained by uniformly mixing each compound containing an active component and water. The amount of water to be used in the slurry liquid is not limited as long as the total amount of the compound to be used can be completely dissolved or uniformly mixed. The amount of water to be used may be appropriately determined in consideration of the drying method and the drying conditions. Usually, the amount of water to be used is 100 parts by mass or more and 2000 parts by mass or less with respect to 100 parts by mass of the total mass of the compound for preparing a slurry. The amount of water may be large, but too large amount of water causes many disadvantages such as an increase in the energy cost of the drying step and a possible failure to completely dry.

The slurry liquid of the source compound of the above each component element is preferably prepared by (a) a method of mixing each of the above source compounds at once, (b) a method of mixing the above source compounds at once and then performing aging, (c) a method of mixing the above source compounds stepwise, (d) a method of repeating mixing step and aging step stepwise, and (e) a method combining (a) to (d). Here, the above aging means "an operation in which industrial raw materials or semi-finished products are processed under specific conditions such as a certain period of time and a certain temperature to conduct acquisition or improvement of the required physical properties and chemical properties, or proceeding of a predetermined reaction". In the present invention, the above certain period of time means a range of 5 minutes or longer and 24 hours or shorter, and the above certain temperature means a range from room temperature to a point equal to or lower than a boiling point of an aqueous solution or an aqueous dispersion liquid. Among these, in terms of the activity and yield of the finally obtained catalyst, preferred is the (c) method of mixing the above source compounds stepwise, more preferred is a method in which each raw material to be mixed with a mother liquid stepwise is completely dissolved to be a solution, and most preferred is a method of mixing various mixed solutions of alkali metal solution and nitrate with a mother liquid in which the raw material of the molybdenum is a mixed solution or slurry. However, it is not always necessary to mix all the elements constituting the catalyst in this step, and some elements or some amounts thereof may be added in the subsequent steps.

In the present invention, the shape of the stirring blade of the stirrer used in mixing the essential active components is not limited. Any stirring blade such as a propeller blade, a turbine blade, a paddle blade, an inclined paddle blade, a screw blade, an anchor blade, a ribbon blade, a large lattice blade can be used in one stage or in two or more stages of which blades are the same or different types in the vertical direction. In addition, a baffle (obstruction plate) may be installed in the reaction tank if necessary.

Then, the slurry liquid thus-obtained is dried. The drying method is not limited so long as the slurry liquid can be completely dried by the method, but examples thereof include drum drying, freeze drying, spray drying and evaporation drying. Among these, spray drying, which allows the slurry liquid to be dried into a powder or granule within a short period of time, is particularly preferred in the present invention. The temperature of the drying with spray drying varies depending on the concentration of the slurry liquid, the liquid sending speed, or the like. Typically, the temperature at the outlet of a drying machine is 70°C or higher and 150°C or lower.

Subjecting the catalyst precursor obtained as described above to preliminary calcination, molding, and then main calcination allows for controlling and holding the obtained shape, and obtaining a catalyst having particularly excellent mechanical strength for industrial use, and the catalyst can exhibit stable catalyst performance.

As for the molding, either a carrying shaping in which the preliminarily calcined powder is carried on a carrier such as silica or a non-carrying shaping in which no carrier is used can be adopted. Specific examples of the molding method include tablet molding, press molding, extrusion molding and granulation molding. As the shape of the molded product, for example, a columnar shape, a ring shape, a spherical shape or the like can be appropriately selected in consideration of operating conditions. Preferred is a carried catalyst in which a catalytically active component is carried on a spherical carrier, particularly an inert carrier such as silica or alumina and in which the average particle size is 3.0 mm or more and 10.0 mm or less, and preferably 3.0 mm or more and 8.0 mm or less. As for the carrying method, a tumbling granulation method, a method using a centrifugal flow coating apparatus, a wash coating method, and the like are widely known. The method is not limited as long as the preliminarily calcined powder can be uniformly carried on the carrier, but the tumbling granulation method is preferred in consideration of the production efficiency of the catalyst and the like. Specifically, the tumbling granulation method is a method in which using a device that has a flat or uneven disk at the bottom of a fixed cylindrical container, a carrier charged into the container is vigorously agitated by means of a repeat of rotation motion and revolution motion of the carrier itself by rotating the disk at a high speed, and then the preliminarily calcined powder is added into the container to carry the powder component on the carrier.

On the occasion of carrying, it is preferred to use a binder. Specific examples of the binder which can be used include water, ethanol, methanol, propanol, a polyhydric alcohol, polyvinyl alcohol as a polymer-based binder and a silica sol aqueous solution as an inorganic binder; ethanol, methanol, propanol, and a polyhydric alcohol are preferred; a diol such as ethylene glycol and a triol such as glycerin is more preferred; and an aqueous solution of glycerin having a concentration of 5 mass% or more is still more preferred. Using an appropriate amount of the glycerin aqueous solution allows for obtaining a high-performance catalyst having good molding properties and high mechanical strength. The amount of these binders to be used is usually 2 to 60 parts by mass with respect to 100 parts by mass of the preliminarily calcined powder, and the amount of the glycerin aqueous solution is preferably 15 to 50 parts by mass. The binder and the preliminarily calcined powder may be alternately supplied to a molding machine or simultaneously supplied to the molding machine on the occasion of the carrying. Further, on the occasion of molding, a small amount of known additives such as graphite and talc may be added. None of a molding aid, a pore-forming agent and a carrier added in the molding is considered as the constituent element of the active component in the present invention, regardless of whether the molding aid, the pore-forming agent and the carrier have the activity in the sense of converting the raw material into some other product.

The preliminary calcination method, the preliminary calcination conditions, the main calcination method, and the main calcination conditions are not limited, but known treatment methods and conditions can be applied. The preliminary calcination or the main calcination is usually carried out at 200°C or higher and 600°C or lower, and preferably 300°C or higher and 550°C or lower, for 0.5 hours or longer, and preferably 1 hour or longer and 40 hours or shorter under the conditions that an oxygen-containing gas such as air or an inert gas flow. Here, the inert gas refers to a gas that does not reduce the reaction activity of the catalyst, and specific examples thereof include nitrogen, carbon dioxide, helium and argon. The optimum conditions for the main calcination vary depending on the reaction conditions when an unsaturated aldehyde and/or an unsaturated carboxylic acid are produced using a catalyst, and changing the process parameters of the main calcination step, that is, the oxygen content in the atmosphere, the maximum temperature reached and the calcination time falls within the scope of the present invention, because the changing is well-known for the skilled person. The main calcination step shall be carried out after the above preliminary calcination step, and the maximum temperature reached (main calcination temperature) in the main calcination step shall be higher than the maximum temperature reached (preliminary calcination temperature) in the above preliminary calcination step. The technique of the calcination includes but not limited to a fluidized bed, rotary kiln, muffle furnace, and tunnel firing furnace, and should be selected within an appropriate range in consideration of the final catalyst performance, mechanical strength, molding properties, production efficiency and the like.

The catalyst of the present invention is preferably used as a catalyst for producing an unsaturated aldehyde compound, an unsaturated carboxylic acid compound, or a conjugated diene compound, is more preferably used as a catalyst for producing an unsaturated aldehyde compound, and is particularly preferably used as a catalyst for producing acrolein from propylene. In a process of an exothermic reaction such as production of an unsaturated aldehyde compound, an unsaturated carboxylic acid compound, or a conjugated diene compound, it is known to those skilled in the art that different catalyst types are filled in multiple layers so that the activity is increased from an inlet side of a reaction tube toward an outlet side of the reaction tube, for the purpose of preventing deterioration of the catalyst itself due to heat generated by the reaction in an actual plant. The catalyst of the present invention can be used on either an inlet side of the reaction tube, an outlet side of the reaction tube, or the middle catalyst layer. For example, the catalyst of the present invention is most preferably used on the most outlet side of the reaction tube, that is, the catalyst of the present invention is used as the most active catalyst among all catalyst layers in the reaction tube. For the multilayer filling, two-layer or three-layer filling is particularly preferred.

Catalyst in Second Stage

When the catalyst of the present invention is used as a catalyst in a first stage, that is, a catalyst for producing an unsaturated aldehyde compound, an unsaturated carboxylic acid compound can be obtained by performing a second-stage oxidation reaction.

In this case, the catalyst of the present invention can also be used as a catalyst in a second stage, but a catalyst containing a catalytically active component represented by the following formula (2) is preferred.

$$Mo_{12}V_{a2}Wb_2Cu_{c2}Sb_{d2}X2_{e2}Y2_{f2}Z2_{g2}O_{h2}... \qquad (2)$$

(In the formula, Mo, V, W, Cu, Sb and O represent molybdenum, vanadium, tungsten, copper, antimony and oxygen, respectively; X2 represents at least one element selected from the group consisting of an alkali metal and thallium; Y2 represents at least one element selected from the group consisting of magnesium, calcium, strontium, barium and zinc; and Z2 represents at least one element selected from the group consisting of niobium, cerium, tin, chromium, manganese, iron, cobalt, samarium, germanium, titanium and arsenic. a2, b2, c2, d2, e2, f2, g2 and h2 represent the atomic proportion of each element, and with respect to molybdenum atom 12, a2 satisfies $0 < a2 \leq 10$, b2 satisfies $0 \leq b2 \leq 10$, c2 satisfies $0 < c2 \leq 6$, d2 satisfies $0 < d2 \leq 10$, e2 satisfies $0 \leq e2 \leq 0.5$, f2 satisfies $0 \leq f2 \leq 1$, and g2 satisfies $0 \leq g2 < 6$. Further, h2 is the number of oxygen atoms required to satisfy the atomic value of each component.)

In the production of a catalyst containing the catalytically active component represented by the above formula (I-2), a method widely known as a method for preparing this kind of a catalyst, for example, an oxide catalyst or a catalyst having a heteropolyacid or a salt structure thereof, can be adopted. The raw materials that can be used in producing the catalyst are not limited, and various materials can be used. For example, molybdenum oxides such as molybdenum trioxide, molybdic acid or salts thereof such as molybdic acid and an ammonium molybdate, molybdenum-containing heteropolyacids or salts thereof such as phosphomolybdic acid and silicomolybdic acid, and the like can be used. The raw material of an antimony component is not limited, but antimony trioxide or antimony acetate is preferred. As raw materials for other elements such as vanadium, tungsten, copper and the like, nitrate, sulfate, carbonate, phosphate, organic acid salt, halide, hydroxide, oxide or the metal of these elements can be used.

A compound containing these active components may be used alone or in combination of two or more.

Next, the slurry liquid obtained above is dried to obtain a solid of catalytically active component. The drying method is not limited so long as the slurry liquid can be completely dried by the method. However, examples thereof include drum drying, freeze drying, spray drying and evaporation drying. Spray drying is preferred because the slurry liquid can be dried into a powder or granule in a short period of time. The temperature of the drying with spray drying varies depending on the concentration of the slurry liquid, the liquid sending speed or the like, but the temperature at the outlet of a drying machine is approximately 70°C to 150°C. In this case, the slurry liquid is preferably dried such that the average particle size of a slurry liquid dried product to be obtained is 10 μm to 700 μm.

The solid of the catalyst precursor in the second stage obtained as described above can be used as it is for a coating mixture, and is preferably subjected to calcination because the molding properties may be improved. The calcination method and the calcination conditions are not limited, and known treatment methods and conditions can be applied. The optimum calcination conditions vary depending on the used raw material for the catalyst, catalyst composition, preparation method and the like. The calcination temperature is usually 100°C to 350°C, preferably 150°C to 300°C, and the calcination time is usually 1 to 20 hours. The calcination is usually carried out in an air atmosphere, but may be carried out in an atmosphere of an inert gas such as nitrogen, carbon dioxide, helium or argon. The calcination in an air atmosphere may be carried out after calcination in an inert gas atmosphere, if necessary. The thus-obtained calcined solid is preferably pulverized before the molding. The pulverizing method is not limited, but it is preferable to use a ball mill.

The compound containing the active component in preparing the slurry for the second stage does not necessarily have to contain all the active components, and a part of the components may be used before the following molding step.

The shape of the catalyst in the second stage is not limited. The catalyst is used by being molded into a columnar shape, a tablet, a ring shape, a spherical shape or the like in order to reduce the pressure loss of a reaction gas in the oxidation reaction. Among these, the solid of catalytically active component is particularly preferably carried on an inert carrier to be a carried catalyst because improvement in selectivity and removal of heat of reaction can be expected. A tumbling granulation method described below is preferred for the carrying. This method is a method in which, for example, in a device that has a flat or uneven disk at the bottom of a fixed container, a carrier in the container is vigorously agitated by repeatedly performing rotation motion and revolution motion by rotating the disk at a high speed, and then a mixture for the carrying including the binder, the solid of catalytically active component and optionally a molding aid and/or a strength improver is carried on the carrier. As a method of adding the binder, any methods may be adapted such as 1) premixing a binder with the mixture for the carrying, 2) adding the binder at the same time as the mixture for the carrying is added into the fixed container, 3) adding the binder after adding the mixture for the carrying into the fixed container, 4) adding the binder before adding the mixture for the carrying into the fixed container, 5) dividedly preparing the mixture for the carrying and the binder independently and adding the whole amount of them in the appropriate combination of 2) to 4). Among these, for example, 5) is preferably performed by adjusting the adding rate using an auto feeder or the like such that the mixture for the carrying does not adhere to the wall of the fixed container and the mixture for the carrying does not aggregate with each other and a predetermined amount of the mixture for the carrying is carried on the carrier. Examples of the binder include water, ethanol, polyhydric alcohol, polyvinyl alcohol as a polymer-based binder, celluloses such as a crystalline cellulose, methyl cellulose and ethyl cellulose, and an aqueous silica sol solution as an inorganic binder. Diols such as cellulose and ethylene glycol and triols such as glycerin are preferred, and an aqueous solution having a concentration of glycerin of 5 mass% or more is particularly preferred. The amount of these binders to be used is usually 2 to 60 parts by mass, preferably 10 to 50 parts by mass, per 100 parts by mass of the mixture for the carrying.

Specific examples of the carrier in the above carrying include a spherical carrier having a diameter of 1 mm to 15 mm, and preferably 2.5 mm to 10 mm, such as silicon carbide, alumina, silica alumina, mullite and arandom. The carriers having a porosity of 10% to 70% are usually used. The carrier and the mixture for the carrying at the ratio of the mixture for the carrying/(mixture for the carrying + carrier) =10 mass% to 75 mass% are usually used, and the carrier and the mixture for the carrying at the ratio of the mixture for the carrying/(mixture for the carrying + carrier) =15 mass% to 60 mass% are preferably used. When the ratio of the mixture for the carrying tends to be large, the reaction activity of the carried catalyst is large, but the mechanical strength tends to be small. On the contrary, when the ratio of the mixture for the carrying is small, the mechanical strength tends to be large, but the reaction activity tends to be small. In the above, examples of the molding aid to be used as necessary include silica gel, diatomite, and alumina powder. The amount of the molding aid to be used is usually 1 to 60 parts by mass with respect to 100 parts by mass of the solid of catalytically active component. If necessary, the use of inorganic fibers (for example, ceramic fibers or whiskers) that are inactive to the solid of catalytically active component and the reaction gas as the strength improver is useful for improving the mechanical strength of the catalyst, and glass fibers are preferred. The amount of the fiber to be used is usually 1 to 30 parts by mass with respect to 100 parts by mass of the solid of catalytically active component. None of a molding aid, a pore-forming agent and a carrier added in the molding of the catalyst for the first stage is considered as the constituent element of the active component in the present invention, regardless of whether the molding aid, the pore-forming agent and the carrier have the activity in the sense of converting the raw material into some other product.

The carried catalyst obtained as described above can be used as a catalyst for the catalytic gas phase oxidation, and is preferably subjected to calcination because the molding properties may be improved. The calcination method and the calcination conditions are not limited, and known treatment methods and conditions can be applied. The optimum calcination conditions vary depending on the raw material for the catalyst to be used, the catalyst composition, the preparation method, and the like, but the calcination temperature is usually 100°C to 450°C, preferably 270°C to 420°C, and the calcination time is usually 1 to 20 hours. The calcination is usually carried out in an air atmosphere, and may be carried out in an atmosphere of an inert gas such as nitrogen, carbon dioxide, helium or argon. The calcination in an air atmosphere may be carried out after the calcination in an inert gas atmosphere, if necessary.

Use of catalyst

When the catalyst according to the present invention is used in a reaction that uses propylene, isobutylene, t-butyl alcohol, butene, or the like as raw materials to produce the corresponding unsaturated aldehyde and unsaturated carboxylic acid, and particularly, in a reaction of producing acrolein and acrylic acid by catalytic gas phase oxidation of propylene with molecular oxygen or a gas containing molecular oxygen, the catalyst allows for improving the catalytic activity and the yield and is very effective in improving the price competitiveness of the product as compared with the known method. In addition, the catalyst is also useful as an oxidation catalyst or an oxidative dehydrogenation catalyst when producing a conjugated diolefin (such as 1,3-butadiene) by a catalytic oxidative dehydrogenation reaction from a mixed gas containing monoolefin (such as butenes) having 4 or more carbon atoms and molecular oxygen.

Moreover, using the catalyst according to the present invention can realize a long-term operation of the catalytic gas phase oxidation method safely, stably, and at a low cost. In addition, the catalyst according to the present invention can be effective in improving the yield even in a region where the catalytic activity is particularly high or in a region where the catalytic activity is not high. Further, the effect of improving the process stability of the partial oxidation reaction accompanied by heat generation such as reduction of $\Delta T$ (difference between hotspot temperature and reaction bath temperature) can be expected. Further, the catalyst according to the present invention is also effective in reducing by-products that adversely influences the environment and the quality of the final product, such as carbon monoxide (CO), carbon dioxide ($CO_2$), acetaldehyde, acetic acid, and formaldehyde.

The thus-obtained catalyst of the present invention can be used, for example, for producing acrolein and/or acrylic acid by catalytic gas phase oxidation of propylene using a molecular oxygen-containing gas. In the production method of the present invention, the method for flowing the raw material gas may be an ordinary single-flow method or a recycling method, and can be carried out under widely used conditions, and is not limited. For example, a mixed gas containing 1 vol% to 10 vol% and preferably 4 vol% to 9 vol% of propylene, 3 vol% to 20 vol% and preferably 4 vol% to 18 vol% of molecular oxygen, 0 vol% to 60 vol% and preferably 4 vol% to 50 vol% of water vapor at room temperature as a starting raw material, and 20 vol% to 80 vol% and preferably 30 vol% to 60 vol% of an inert gas such as carbon dioxide and nitrogen is introduced to the catalyst of the present invention filled in a reaction tube at 250°C to 450°C under normal pressure to 10 atm and a space velocity of 300 to 5000 $h^{-1}$ to perform a reaction.

In the present description, unless otherwise specified, the improvement of the catalytic activity means that the conversion rate of raw materials is high as compared with that of a control, when the catalytic reaction is carried out at the same reaction bath temperature.

In the present invention, unless otherwise specified, a high yield means a high total yield of the corresponding unsaturated aldehyde and/or unsaturated carboxylic acid when the oxidation reaction is carried out using propylene, isobutylene, t-butyl alcohol, or the like as raw materials. In addition, unless otherwise specified, the yield refers to an useful yield described later.

In the present description, unless otherwise specified, the constituent elements of the catalyst precursor refer to all the elements used in the method for producing a catalyst. Raw materials and constituent elements of the raw materials that disappear, sublimate, volatilize, and burn at the maximum temperature or lower in a main calcination step are not included in the constituent elements of the catalyst precursor. Further, silicon and other elements constituting inorganic materials contained in the shaping aid and the carrier are not included as the constituent elements of the catalyst precursor.

In the present invention, the hotspot temperature is the maximum temperature in the temperature distribution in a catalyst-filled bed that is measured by installing thermocouples in the long axis direction in a multi-tube reaction tube, and the reaction bath temperature is a set temperature of a heat medium used for the purpose of cooling the heat generation of the reaction tube. The number of points for measuring the temperature distribution is not limited, but for example, the temperature distribution is measured in a state that the catalyst filling length is evenly divided from 10 to 1,000.

In the present description, the unsaturated aldehyde and the unsaturated aldehyde compound are organic compounds having at least one double bond and at least one aldehyde in the molecule, such as acrolein and methacrolein. In the present invention, the unsaturated carboxylic acid and the unsaturated carboxylic acid compound are organic compounds having at least one double bond and at least one carboxy group or an ester group in the molecule, such as acrylic acid, methacrylic acid, and methyl methacrylate. In the present description, the conjugated diene refers to a diene in which double bonds are separated by one single bond and are chemically conjugated, such as 1,3-butadiene.

EXAMPLES

Hereinafter, the present invention will be described in more detail with reference to Examples. In Examples, a conversion rate, an useful yield, an useful selectivity, and an active mass ratio were calculated according to the following equations.

$$\text{Conversion rate (\%)} = \text{(number of moles of reacted propylene, t-butyl alcohol, isobutylene, or n-butene)/(number of moles of supplied propylene, t-butyl alcohol, isobutylene, or n-butene)} \times 100$$

$$\text{Useful yield (\%)} = \text{(total number of moles of produced acrolein and acrylic acid, total number of moles of produced methacrolein and methacrylic acid, or total number of moles of produced butadiene)/(number of moles of supplied propylene, t-butyl alcohol, isobutylene, or n-butene)} \times 100$$

$$\text{Useful selectivity (\%)} = \text{(total number of moles of produced acrolein and acrylic acid, total number of moles of produced methacrolein and methacrylic acid, or total number of moles of produced butadiene)/(number of moles of reacted propylene, t-butyl alcohol, isobutylene, or n-butene)} \times 100$$

$$\text{Active mass ratio (mass\%)} = \text{(mass of preliminarily calcined powder used for molding)/\{(mass of preliminarily calcined powder used for molding) + (mass of carrier used for molding)\}} \times 100$$

Example 1

(Preparation of catalyst 1)

100 parts by mass of ammonium heptamolybdate was completely dissolved in 380 parts by mass of pure water heated to 60°C (mother liquid 1). Next, 0.27 parts by mass of potassium nitrate was dissolved in 2.4 parts by mass of pure water, and the mixture was added to the mother liquid 1. Next, 40 parts by mass of ferric nitrate, 86 parts by mass of cobalt nitrate, and 30 parts by mass of nickel nitrate were dissolved in 83 parts by mass of pure water heated to 60°C, and the mixture was added to the mother liquid 1. Subsequently, 18 parts by mass of bismuth nitrate was dissolved in an aqueous nitric acid solution prepared by adding 4.7 parts by mass of nitric acid (60 mass%) to 19 parts by mass of pure water heated to 60°C, and the mixture was added to the mother liquid 1. This mother liquid 1 (solid content concentration: 27 mass%) was dried by a spray drying at an inlet temperature of 240°C, an outlet temperature of 120°C, and a rotation speed of an atomizer of 14,000 rpm, and the obtained dry powder was subjected to preliminary calcination at 440°C for 4 hours. 5 mass% of a crystalline cellulose with respect to the thus-obtained preliminarily calcined powder (the atomic ratio calculated based on the charged raw materials was Mo:Bi:Fe:Co:Ni:K = 12:0.8:2.1:6.3:2.2:0.06, the bulk density was 0.88 g/mL) was added to the preliminarily calcined powder, followed by thorough mixing. Then, the mixture was carried and molded into a spherical shape on an inert carrier by using a 33 mass% glycerin solution as a binder by a tumbling granulation method such that the active mass ratio was 50 mass%. The thus-obtained spherical molded product having a particle diameter of 5.3 mm was subjected to main calcination under the conditions of 540°C and 4 hours to obtain a catalyst 1. In the catalyst 1, the catalyst precursor had a D10 of 19 $\mu$m, a D50 of 35 $\mu$m, and a D90 of 59 $\mu$m, and the hollow particle proportion of the catalyst precursor was 1.0%.

Example 2

(Preparation of catalyst 2)

A catalyst 2 was obtained in the same manner as the catalyst 1 in Example 1 except that the spray drying step was performed at an inlet temperature of 240°C, an outlet temperature of 130°C, and a rotation speed of an atomizer of 18,000 rpm. In the catalyst 2, the catalyst precursor had a D10 of 13 $\mu$m, a D50 of 25 $\mu$m, and a D90 of 43 $\mu$m, and the hollow particle proportion of the catalyst precursor was 0.0%.

Example 3

(Preparation of catalyst 3)

A catalyst 3 was obtained in the same manner as the catalyst 1 in Example 1 except that the solid content concentration of the mother liquid 1 was 14 mass% and the spray drying step was performed at an inlet temperature of 240°C, an outlet temperature of 130°C, and a rotation speed of an atomizer of 18,000 rpm. In the catalyst 3, the catalyst precursor had a D10 of 16 $\mu$m, a D50 of 30 $\mu$m, and a D90 of 50 $\mu$m, and the hollow particle proportion of the catalyst precursor was 0.8%.

Example 4

(Preparation of catalyst 4)

A catalyst 4 was obtained in the same manner as the catalyst 1 in Example 1 except that the solid content concentration of the mother liquid 1 was 14 mass% and the spray drying step was performed at an inlet temperature of 240°C, an outlet temperature of 130°C, and a rotation speed of an atomizer of 22,000 rpm. In the catalyst 4, the catalyst precursor had a D10 of 14 $\mu$m, a D50 of 24 $\mu$m, and a D90 of 40 $\mu$m, and the hollow particle proportion of the catalyst precursor was 0.0%.

Example 5

(Preparation of catalyst 5)

100 parts by mass of ammonium heptamolybdate was completely dissolved in 380 parts by mass of pure water heated to 60°C (mother liquid 1). Next, 0.37 parts by mass of potassium nitrate was dissolved in 3.5 parts by mass of pure water, and the mixture was added to the mother liquid 1. Next, 34 parts by mass of ferric nitrate, 81 parts by mass of cobalt nitrate, and 44 parts by mass of nickel nitrate were dissolved in 82 parts by mass of pure water heated to 60°C, and the mixture was added to the mother liquid 1. Subsequently, 21 parts by mass of bismuth nitrate was dissolved in an aqueous nitric acid solution prepared by adding 5.8 parts by mass of nitric acid (60 mass%) to 24 parts by mass of pure water heated to 60°C, and the mixture was added to the mother liquid 1. This mother liquid 1 (solid content concentration: 27 mass%) was dried by a spray drying at an inlet temperature of 300°C, an outlet temperature of 110°C, and a rotation speed of an atomizer of 18,000 rpm, and the obtained dry powder was subjected to preliminary calcination at 440°C for 4 hours. 5 mass% of a crystalline cellulose with respect to the thus-obtained preliminarily calcined powder (the atomic ratio calculated based on the charged raw materials was Mo:Bi:Fe:Co:Ni:K = 12:0.9:1.8:5.9:3.2:0.08, the bulk density was 1.06 g/mL) was added to the preliminarily calcined powder, followed by thorough mixing. Then, the mixture was carried and molded into a spherical shape on an inert carrier by using a 33 mass% glycerin solution as a binder by a tumbling granulation method such that the active mass ratio was 50 mass%. The thus-obtained spherical molded product having a particle diameter of 5.3 mm was subjected to main calcination under the conditions of 510°C and 4 hours to obtain a catalyst 5. In the catalyst 5, the catalyst precursor had a D10 of 16 μm, a D50 of 31 μm, and a D90 of 55 μm, and the hollow particle proportion of the catalyst precursor was 2.3%.

Example 6

(Preparation of catalyst 6)

A catalyst 6 was obtained in the same manner as the catalyst 1 in Example 5 except that the spray drying step was performed at an inlet temperature of 240°C, an outlet temperature of 110°C, and a rotation speed of an atomizer of 14,000 rpm. In the catalyst 6, the catalyst precursor had a D10 of 19 μm, a D50 of 37 μm, and a D90 of 64 μm, and the hollow particle proportion of the catalyst precursor was 2.3%.

Comparative Example 1

(Preparation of catalyst 7)

A catalyst 7 was obtained in the same manner as the catalyst 1 in Example 1 except that the spray drying step was performed at an inlet temperature of 300°C, an outlet temperature of 95°C, and a rotation speed of an atomizer of 14,000 rpm. In the catalyst 7, the catalyst precursor had a D10 of 26 μm, a D50 of 42 μm, and a D90 of 66 μm, and the hollow particle proportion of the catalyst precursor was 4.8%.

Comparative Example 2

(Preparation of catalyst 8)

A catalyst 8 was obtained in the same manner as the catalyst 1 in Example 1 except that the solid content concentration of the mother liquid 1 was 55 mass% and the spray drying step was performed at an inlet temperature of 300°C, an outlet temperature of 95°C, and a rotation speed of an atomizer of 14,000 rpm. In the catalyst 8, the catalyst precursor had a D10 of 25 μm, a D50 of 43 μm, and a D90 of 72 μm, and the hollow particle proportion of the catalyst precursor was 4.5%.

Comparative Example 3

(Preparation of catalyst 9)

100 parts by mass of ammonium heptamolybdate was completely dissolved in 380 parts by mass of pure water heated to 60°C (mother liquid 1). Next, 0.45 parts by mass of potassium nitrate was dissolved in 45 parts by mass of pure water and added to the mother liquid 1. Next, 38 parts by mass of ferric nitrate, 71 parts by mass of cobalt nitrate, and 38 parts by mass of nickel nitrate were dissolved in 76 parts by mass of pure water heated to 60°C, and the mixture was added to the mother liquid 1. Subsequently, 36 parts by mass of bismuth nitrate was dissolved in an aqueous nitric acid solution prepared by adding 9.7 parts by mass of nitric acid (60 mass%) to 41 parts by mass of pure water heated to 60°C, and the mixture was added to the mother liquid 1. This mother liquid 1 was dried by a spray drying at an inlet temperature of 240°C, an outlet temperature of 110°C, and a rotation speed of an atomizer of 14,000 rpm, and the obtained dry powder was subjected to preliminary calcination at 440°C for 4 hours. 5 mass% of a crystalline cellulose with respect to the thus-obtained preliminarily calcined powder (the atomic ratio calculated based on the charged raw materials was Mo:Bi:Fe:Co:Ni:K = 12:1.6:2.0:5.2:2.8:0.10) was added to the preliminarily calcined powder, followed by thorough mixing. Then, the mixture was carried and molded into a spherical shape on an inert carrier by using a 33 mass% glycerin solution as a binder by a tumbling granulation method such that the active mass ratio was 50 mass%. The thus-obtained spherical molded product having a particle diameter of 5.3 mm was subjected to main calcination under the conditions of 530°C and 4 hours to obtain a catalyst 9.

Using the catalyst 1 to the catalyst 8, the oxidation reaction of propylene was carried out by the following method, and the useful yield was determined. 25 mL of the catalyst 9 was charged into a gas inlet side of a stainless steel reaction tube having an inner diameter of 28.4 mm, and the catalyst 1 to the catalyst 8 were separately charged in an amount of 45 mL into a gas outlet side of the stainless steel reaction tube, and a mixed gas having a gas volume ratio of propylene:oxygen:water vapor:nitrogen = 1.0:1.7:1.3:9.5 was introduced at a propylene space velocity of 100 hr$^{-1}$ with respect to all the catalysts in the reaction tube to carry out the oxidation reaction of propylene. After the aging reaction lasted for 20 hours or longer from the start of the reaction at a reaction bath temperature of 315°C, the useful yield (UY) at a conversion rate of raw materials of 97.5% interpolation shown in Table 1 was obtained by analyzing the gas at the outlet of the reaction tube at a reaction bath temperature of about 320°C. Results are shown in Table 1. Table 1 shows the inlet temperature, the outlet temperature, the rotation speed of atomizer, the relative centrifugal acceleration, the solid content concentration in the mother liquid 1, and the parameter SD in the spray drying step during the production of the catalyst precursor for each catalyst. Table 1 also shows the D10, the D50, the D90, the specific surface area, and the hollow particle proportion of the catalyst precursor for each catalyst. The D10, the D50, the D90, and the specific surface area, shown in Table 1 were evaluated by the methods exemplified in the embodiments.

Table 1

| Catalyst | Inlet temperature /°C | Outlet temperature /°C | Rotation speed of atomizer /rpm | Relative centrifugal acceleration /G | Solid content concentration /mass% | SD | D10 /μm | D50 /μm | D90 /μm | Specific surface area of catalyst precursor /m²/g | Hollow particle proportion of catalyst precursor /% | UY /% |
|---|---|---|---|---|---|---|---|---|---|---|---|---|
| Catalyst 1 | 240 | 120 | 14,000 | 12,052 | 27 | 36 | 19 | 35 | 59 | 7.6 | 1.0 | 91.6 |
| Catalyst 2 | 240 | 130 | 18,000 | 19,923 | 27 | 29 | 13 | 25 | 43 | 100 | 0.0 | 92.1 |
| Catalyst 3 | 240 | 130 | 18,000 | 19,923 | 14 | 29 | 16 | 30 | 50 | 9.9 | 0.8 | 91.8 |
| Catalyst 4 | 240 | 130 | 22,000 | 29,761 | 14 | 22 | 14 | 24 | 40 | 9.7 | 0.0 | 92.0 |
| Catalyst 5 | 300 | 110 | 18,000 | 19,923 | 27 | 35 | 16 | 31 | 55 | 9.2 | 2.2 | 92.3 |
| Catalyst 6 | 240 | 110 | 14,000 | 12,052 | 27 | 37 | 19 | 37 | 64 | 10.1 | 2.3 | 92.0 |
| Catalyst 7 | 300 | 95 | 14,000 | 12,052 | 27 | 43 | 26 | 42 | 66 | 10.5 | 4.8 | 91.5 |
| Catalyst 8 | 300 | 95 | 14,000 | 12,052 | 55 | 43 | 25 | 43 | 72 | 10.9 | 4.5 | 91.3 |

As seen from the above results, with the catalyst prepared from the catalyst precursor according to the present invention, target compounds, i.e., acrolein and acrylic acid, can be obtained in a higher yield than catalysts in the related art.

Although the present invention has been described in detail with reference to specific examples, it is clear to those skilled in the art that various alterations and modifications can be added without departing from the spirit and the scope of the present invention.

The present application is based on a Japanese patent application (Japanese Patent Application No. 2020-159209) filed on September 24, 2020, the entire contents of which are incorporated herein by reference. In addition, all references referred herein are entirely incorporated.

INDUSTRIAL APPLICABILITY

When the catalyst according to the present invention is used to oxidatively produce an unsaturated aldehyde compound, an unsaturated carboxylic acid compound, or a conjugated diene compound, the target compound can be obtained in a high yield.

**Claims**

1. A catalyst precursor represented by the following formula (1) having an average particle diameter (D50), which is a particle diameter at which a cumulative volume fraction is 50%, of 10 $\mu$m or more and 40 $\mu$m or less:

$$Mo_{a1}Bi_{b1}Ni_{c1}Co_{d1}Fe_{e1}X_{f1}Y_{g1}Z_{h1}O_{i1} \qquad (1)$$

where Mo, Bi, Ni, Co and Fe represent molybdenum, bismuth, nickel, cobalt, and iron, respectively; X is at least one element selected from tungsten, antimony, tin, zinc, chromium, manganese, magnesium, calcium, silicon, aluminum, cerium, and titanium; Y is at least one element selected from sodium, potassium, cesium, rubidium, and thallium; Z belongs to the 1st to 16th groups in the periodic table and represents at least one element selected from elements other than the above Mo, Bi, Ni, Co, Fe, X, and Y; a1, b1, c1, d1, e1, f1 g1, h1, and i1 represent the number of atoms of molybdenum, bismuth, nickel, cobalt, iron, X, Y, Z, and oxygen, respectively; when a1 = 12, $0 < b1 \leq 7$, $0 \leq c1 \leq 10$, $0 < d1 \leq 10$, $0 < c1+d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element.

2. The catalyst precursor according to claim 1, wherein a hollow particle proportion is 0.0% or more and 4.3% or less.

3. The catalyst precursor according to claim 1 or 2, wherein a specific surface area is 5.0 $m^2$/g or more and 10.4 $m^2$/g or less.

4. A catalyst obtained by molding the catalyst precursor according to any one of claims 1 to 3.

5. A catalyst in which the catalyst precursor according to any one of claims 1 to 3 is carried on an inert carrier.

6. The catalyst according to claim 5, wherein the inert carrier is silica and/or alumina.

7. The catalyst precursor or the catalyst according to any one of claims 1 to 6, wherein the catalyst precursor or the catalyst is used for producing an unsaturated aldehyde compound and/or an unsaturated carboxylic acid compound.

8. A method for producing an unsaturated aldehyde compound and/or an unsaturated carboxylic acid compound comprising using the catalyst precursor or the catalyst according to any one of claims 1 to 7.

9. The method for producing an unsaturated aldehyde compound and/or an unsaturated carboxylic acid compound according to claim 8, wherein the unsaturated aldehyde compound is acrolein and the unsaturated carboxylic acid compound is acrylic acid.

10. A method for producing a catalyst comprising using the catalyst precursor according to any one of claims 1 to 3.

## FIG. 1

## FIG. 2

## FIG. 3

# FIG. 4

## INTERNATIONAL SEARCH REPORT

International application No.

**PCT/JP2021/033592**

### A. CLASSIFICATION OF SUBJECT MATTER

*B01J 23/887*(2006.01)i; *B01J 37/00*(2006.01)i; *C07B 61/00*(2006.01)i; *C07C 27/14*(2006.01)i; *C07C 45/34*(2006.01)i; *C07C 47/22*(2006.01)i; *C07C 51/25*(2006.01)i; *C07C 57/05*(2006.01)i
FI: B01J23/887 Z; B01J37/00 F; C07C27/14 A; C07C47/22 A; C07C45/34; C07C51/25; C07C57/05; C07B61/00 300

According to International Patent Classification (IPC) or to both national classification and IPC

### B. FIELDS SEARCHED

Minimum documentation searched (classification system followed by classification symbols)

B01J21/00-38/74; C07B61/00; C07C27/14; C07C45/34; C07C47/22; C07C51/25; C07C57/05

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2018-521851 A (EVONIK DEGUSSA GMBH) 09 August 2018 (2018-08-09) claims, paragraphs [0061]-[0082], examples | 1, 3-10 |
| A | | 2 |
| X | JP 11-239724 A (MITSUBISHI RAYON CO., LTD.) 07 September 1999 (1999-09-07) claim 1, paragraph [0010], example 2 | 1, 3, 4, 7-10 |
| A | | 2, 5, 6 |
| X | JP 2010-515564 A (BASF SE) 13 May 2010 (2010-05-13) paragraphs [0001], [0036], [0037], examples | 1, 3-10 |
| A | | 2 |
| A | JP 2003-93882 A (MITSUBISHI RAYON CO., LTD.) 02 April 2003 (2003-04-02) claim 1, paragraphs [0018], [0019], examples 1-4 | 1-10 |
| P, X | JP 2020-157294 A (NIPPON KAYAKU CO., LTD.) 01 October 2020 (2020-10-01) claims, paragraphs [0028], [0029], [0031], examples 7, 8, table 1 | 1, 3-10 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 October 2021** | **09 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/033592**

| Patent document cited in search report | | | Publication date (day/month/year) | Patent family member(s) | | | Publication date (day/month/year) |
|---|---|---|---|---|---|---|---|
| JP | 2018-521851 | A | 09 August 2018 | US | 2018/0207617 | A1 | |
| | | | | claims, paragraphs [0070]-[0117], examples | | | |
| | | | | WO | 2017/013115 | A1 | |
| | | | | EP | 3325148 | A1 | |
| | | | | EP | 3120926 | A1 | |
| | | | | CN | 107847922 | A | |
| JP | 11-239724 | A | 07 September 1999 | (Family: none) | | | |
| JP | 2010-515564 | A | 13 May 2010 | US | 2008/0171897 | A1 | |
| | | | | paragraphs [0001], [0079], [0080], examples | | | |
| | | | | WO | 2008/087115 | A2 | |
| | | | | EP | 2104646 | A2 | |
| | | | | CN | 101583569 | A | |
| | | | | KR | 10-2009-0104104 | A | |
| JP | 2003-93882 | A | 02 April 2003 | (Family: none) | | | |
| JP | 2020-157294 | A | 01 October 2020 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016136882 A
- JP 2017024009 A
- JP 6621569 B
- JP 6204862 B
- JP 2020159209 A